# EUROPEAN PATENT SPECIFICATION

(11) **EP 1 476 069 B1**
(45) Date of publication and mention of the grant of the patent: **18.11.2015**
(21) Application number: 02784288.9
(22) Date of filing: 25.10.2002
(51) Int. Cl.: A61B 10/00

(54) **BONE BIOPSY INSTRUMENT HAVING IMPROVED SAMPLE RETENTION**
KNOCHENBIOPSIEINSTRUMENT MIT VERBESSERTER PROBENRETENTION
INSTRUMENT DE BIOPSIE MEDULLAIRE A RETENTION AMELIOREE DE L'ECHANTILLON

(30) Priority: 25.10.2001 US 335694 P
(43) Date of publication of application: 17.11.2004
(73) Proprietor: CareFusion 2200, Inc., San Diego, CA 92130 (US)
(72) Inventor: KRUEGER, John, A., Milwaukee, WI 53222 (US)
(74) Representative: Potter Clarkson LLP
(86) International application number: PCT/US2002/034253
(87) International publication number: WO 2003/034915

(56) References cited:
- EP-A- 1 136 039
- WO-A-00/10465
- US-A- 3 929 123
- US-A- 5 477 862
- US-A- 5 615 690
- US-A- 5 871 453
- US-A- 5 885 226
- US-A- 6 086 543
- US-A- 6 110 128

## Description

### FIELD OF THE INVENTION

The invention relates to the field of medical devices for use in biopsy procedures. In particular, the invention pertains to a bone marrow biopsy device for obtaining bone marrow samples.

### BACKGROUND OF THE INVENTION

Biopsy samples from bone tissue are typically collected from a sampling site in a patient by the use of bone biopsy devices. Typical bone biopsy devices include a hollow cannula which surrounds a stylet The style includes a sharp distal tip which extends distaly beyond the tip of the hollow cannula when the stylet is secured within the cannula. The combined cannula and stylet is used to penetrate through the cortex or outer layer of bone so as to sample the softer tissue or marrow within the bone. Once the cannula and stylet have penetrated into the bone, the stylet is removed and the cannula further advanced into the bone to capture a marrow sample.

The architecture of the tissue sample is important in several respects. Initially, the size of the sample is important, with larger sample sizes representing better samples for subsequent testing to be performed on the tissue. The larger the cannula and stylet which is used, however, the more pain is generatec at the penetration site for the patient. Another aspect of sampling is minimizing damage to the sample, such as compressive forces, during sampling and removal.

A variety of bone biopsy devices have been proposed to improve the biopsy sampling procedure. Andelin et al. U.S. Patent No. 6,110,128, Guirtino et al. U.S. Patent No. 5,615,690 and Mittermeier et al. U.S. Patent No. 6,063,037 describe biopsy devices with structural features designed to enhance sample retention. Other bone biopsy devices have been developed which aid in the preservation of sample integrity by virtue of their structure. One such device is described in Krueger et al. , U. S. Patent No. 6,443, 910, which includes a sampling cannula having a "cutting finger" on the distal portion of the cannula.

EP-A-1,136,039 discloses a bone marrow biopsy assembly that comprises an outer cannula or biopsy needle and a biopsy extractor that is sized for insertion into the proximal end of the outer cannula. This document discloses the features defined in the preamble of claim 1.

US-5,871,453 discloses a biopsy sampling device having an elongated flexible instrument that is provided with cutting jaws at its distal end for removing tissue samples.

US-5,615,690 discloses a biopsy cannula having a tube member with a distal end that lies in two planes. The cannula can be used with a stylet.

US-5,885,226 discloses a bone marrow biopsy needle comprising a tube that is equipped with a cutting means at its distal end.

WO 00/10465 discloses a bone marrow biopsy needle comprising an outer cannula, an inner cannula slidable within the outer cannula, an obturator and an ejector rod. The inner cannula has a tissue receiving region at its distal end.

US-6,110,128 discloses a bone marrow biopsy needle comprising a cannula and a stylet.

Difficulty has been encountered in the art in the balancing between the structural requirements of bone biopsy devices and desirable sampling attributes. Providing bone biopsy devices that consistently sample without damaging forces being exerted upon the sample has proven challenging. Furthermore, accommodating patient comfort by reducing the need for multiple site sampling has presented another challenge. Preserving the architecture of the sample during its obtaining and removal presents yet another factor to be balanced in bone biopsy devices.

There is a need in the field of medical bone biopsy devices for biopsy devices which facilitate the retention of the obtained sample while at the same time preserving the structural integrity of the sample and reducing the amount of trauma to the patient.

### SUMMARY OF THE INVENTION

The invention provides for a bone marrow biopsy system comprising a sampling cannula, having structural features which improve the ability to sever and retain a relatively large marrow sample. It has been discovered that a sampling cannula can be constructed which affords the benefits of obtaining a relatively long core of bone tissue sample and enhancing the retention of the sample within the cannula while at the same time preserving the structural integrity of the sample. In particular, it has been discovered that a bone marrow sampling cannula having the advantages of an open trough-like distal structure can comprise both a friction-enhancing interior surface texture and wall openings which facilitate sample retention without substantially damaging the biological "architecture" of the core sample.

According, the present invention provides a bone marrow biopsy system according to claim 1.

In a preferred embodiment, the trough portion comprises a plurality of wall openings. In another preferred embodiment, each wall opening comprise a substantially rectangular shape.

In an embodiment, the bone marrow biopsy system comprises a stylet structured to be removably inserted into the outer cannula and the outer cannula has a sharpened distal tip;

In a preferred embodiment, the distal tip of the trough portion of the sampling cannula resides within the outer cannula such that the trough portion distal tip terminates proximal to the distal tip of the outer cannula. The bone marrow biopsy system can further comprise an ejector rod.

### BRIEF DESCRIPTION OF THE DRAWINGS

**Figure 1** is an overall perspective viewofthe sampling cannula and an ejector rod according to one embodiment of the invention.
**Figure 2** is a side view of the sampling cannula according to one embodiment of the invention.
**Figure 3** is an enlarged frontal view of the distal trough portion of the sampling cannula according to one embodiment of the invention.
**Figure 4** is a disassembled view of a bone marrow biopsy system comprising a sampling cannula, outer cannula and stylet according to one embodiment of the invention.
**Figure 5** is an illustration of a sequence of biopsy steps with a bone biopsy system comprising the sampling cannula according to one embodiment of the invention.

### DETAILED DESCRIPTION OF THE DRAWINGS

As used herein, the terms "trough" and "trough-like" as used to describe a structural feature of the device of the invention, are meant to describe a cannula structure having an open segment at which the interior surface of the cannula is exposed through an elongated, generally linear open region on the opposite side from an intact portion.

The term "substantially rectangular" as used to refer to a wall opening of the sampling cannula is intended to encompass variations of length, width, and overall shape provided there is an overall longitudinal dimension of such opening.

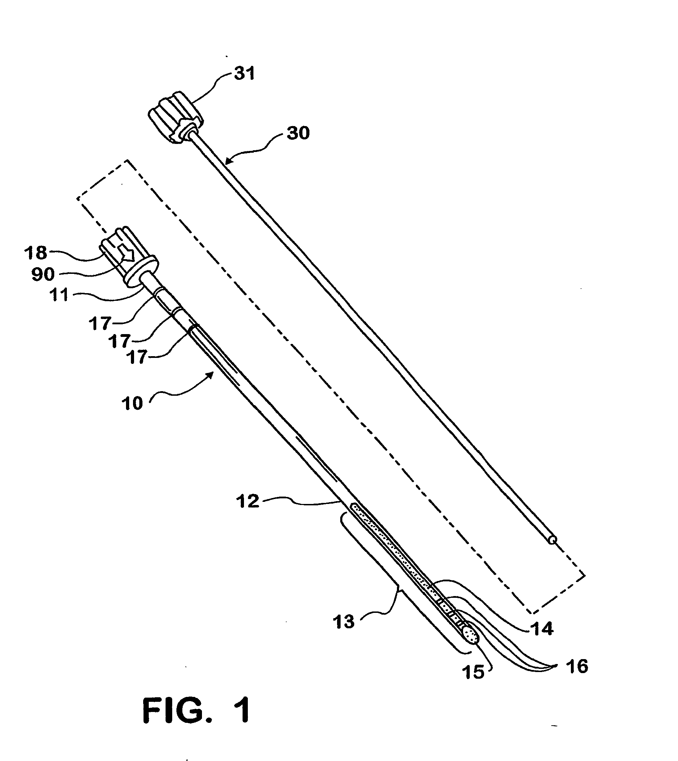

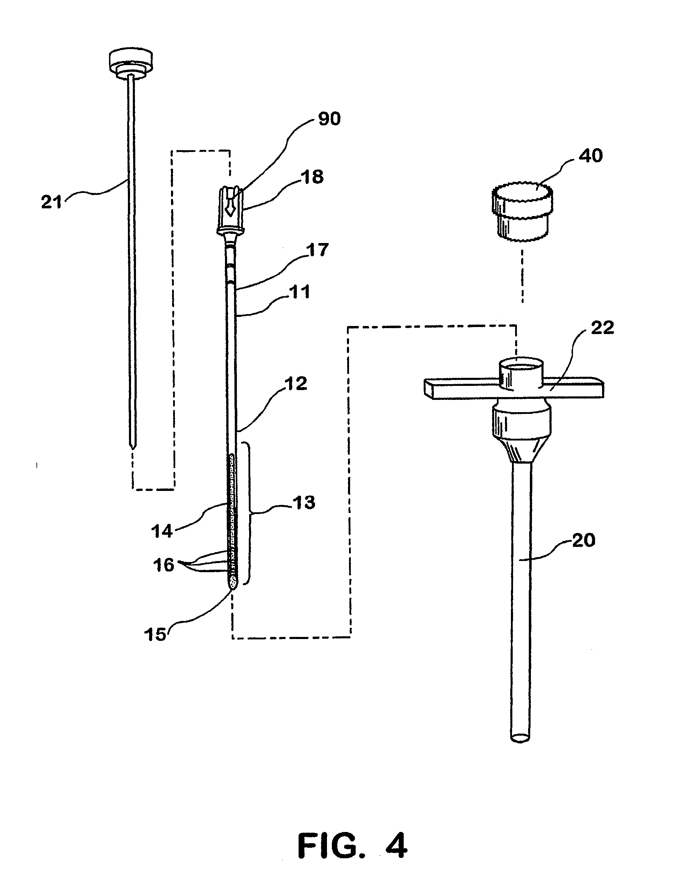

Referring to Figures 1, 2 and 3, the sampling cannula of the invention generally comprises a cannula body 10 structure having a longitudinal body with proximal and distal portions 11 and 12 respectively. The distal portion 12 of the sampling cannula comprises a trough portion 13 in which the cannula 10 structure is circumferentially incomplete, creating an exposed interior surface 14 of the sampling cannula viewable through an elongated open portion on the opposite side from an intact portion. The trough portion 13 originates at a location proximal to the distal tip and extends to terminate at the distal tip 15 of the cannula body 10. The sampling cannula, by way of the trough portion 13, is adapted to rotate about its longitudinal axis in order to sever a sample from the sampling site when positioned within an outer cannula 20 (see Figure 4) which is structured to "core" into the bone tissue. The sampling cannula can be rotated 360° or less as needed to sever the sample from surrounding tissue. With the sample residing within the trough portion 13, the sampling cannula with the sample therein is withdrawn from the site. The wall opening(s) and friction-enhancing interior surface of the trough portion 13 facilitate retention of the sample. Accordingly, the amount of compressive force exerted upon the sample is significantly reduced during separation of the sample from surrounding tissue) and retaining the sample. Thus, damage to the sample and altering its in situ "architecture" as a result of compression is reduced or avoided.

The dimensions of the cannula 10 and trough 13 can vary according to the nature of the sampling site and/or desired sample size. The cannula cross-sectional diameter of the device can vary provided the device can effectively obtain and retain a sample within. For example, 2.7 mm, 3.7 mm or 4.3 mm (8 gauge, 11 gauge or 13 gauge) sizes can be used. For a given combination of dimensions and materials to be used, the hoop strength of the trough portion, i.e., structural integrity of the intact cross-sectional circumference of the intact portion of the cannula body, must be maintained to an extent sufficient to withstand the physical forces exerted upon it during the penetration and sampling stages of the bone marrow biopsy procedure.

The length of the trough portion 13 can vary according to the sample size desired provided the structural integrity of the device is not adversely compromised when sampling forces are exerted upon it. In a preferred embodiment, the trough portion 13 can have a length of up to about 4 cm. Most preferably, the length of the trough portion 13 is about 3 cm. A relatively long trough length is preferred so as to permit a lengthier core sample to be obtained from a patient. Such lengthy sample sizes allow the user to advantageously observe the pathological history of the sampled bone.

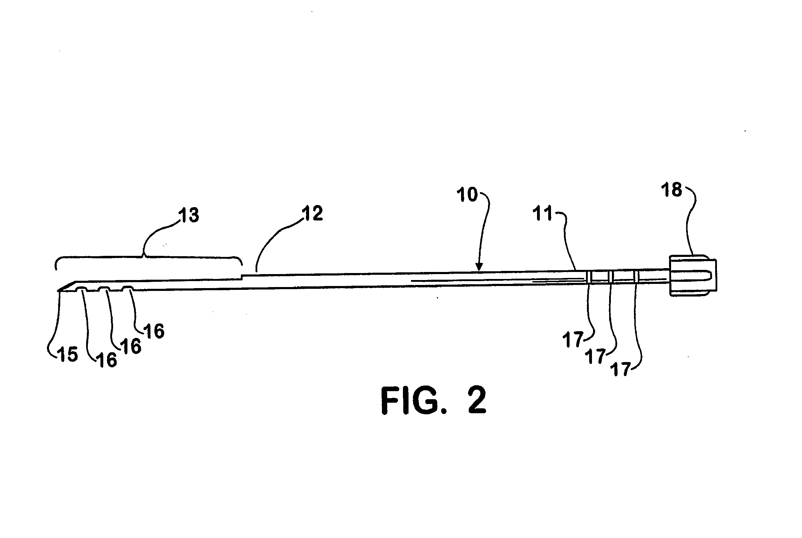

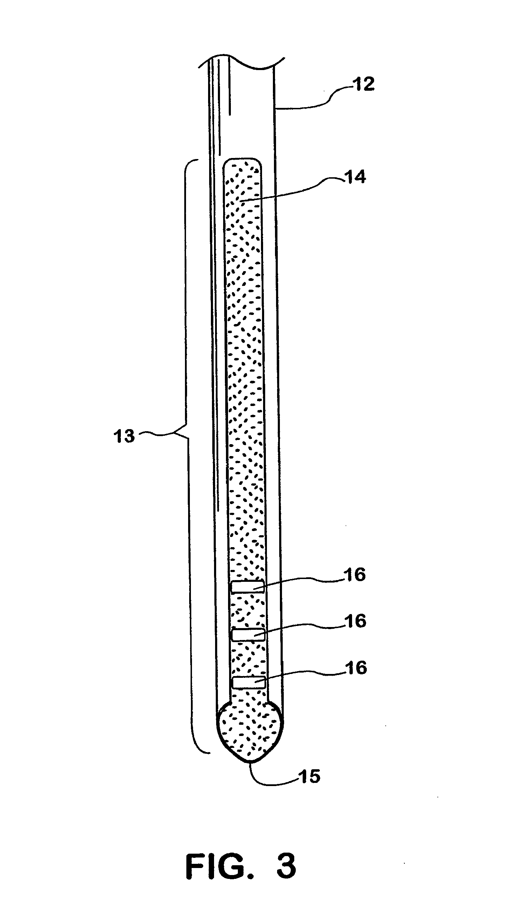

The distal tip 15 of the trough portion 13 of the sampling cannula 10 can be shaped to facilitate penetration and cutting of the bone tissue. In one embodiment and as shown in Figure 2, the tip is angled relative to the longitudinal axis of the cannula and comprises a distal cutting edge which is beveled or shaped into a rounded curve as shown in Figures 1 and 3. In a preferred embodiment, the overall length of the sampling cannula is such that when positioned within the outer cannula, the distal cutting tip 15 of the sampling cannula resides within the outer cannula proximal to the distal tip of the outer cannula.

The dimensions of the trough portion 13 in terms of cross-sectional intact circumference of the cannula body 10 can vary provided sampling by rotational motion thereof can effectively sever and retain a sample therein without adversely compromising the structural integrity of the trough portion. In addition to trough portion length, the rigidity of the cannula body material, thickness of the cannula wall, diameter (gauge) of the cannula all must be balanced together with the amount of the intact portion of the cannula body in the trough. The amount of intact cross-sectional circumference of the trough portion ranges from about 65% to about 85% of a complete cannula circumference. The amount of the intact portion of the trough will vary in cooperation with the diameter or size of the cannula used. Accordingly, the larger the diameter (or lower the gauge) of the cannula, the greater the amount of intact portion required. For example, if a 4.3 mm (13 gauge) cannula is used for the device, the amount of intact circumferential portion can be about 65%, whereas is an 2.7 mm (8 gauge) cannula is used, the amount of intact circumferential portion can be about 85%.

During sampling, opening(s) 16 through the wall of the trough portion function to permit a slight encroachment of the tissue therein, thereby physically "interlocking" or engaging a portion of the sample within and providing resistance to longitudinal migration of the sample. The number, size, shape and arrangement of wall opening(s) 16 in the trough portion 13 can vary provided the opening(s) 16 enhance the retention of the sample within the trough. In accordance with the invention, at least one opening 16 is present through the wall of the trough portion 13. In a preferred embodiment, a plurality of wall openings 16 in the trough portion 13 are present. In one embodiment shown in the Figures, three openings can be present.

The shape of the wall opening(s) 16 can vary as well. Opening shapes which can be used include, but are not limited to, rectangular, square, ovular, circular, triangular, and the like. A preferred opening shape is substantially rectangular opening wherein the longer dimension is perpendicular to the longitudinal axis of the sampling cannula body as shown in Figure 3, illustrating a plurality of substantially rectangular openings.

The size of opening 16, i.e., opening dimensions, can vary provided it is large enough to permit encroachment of the collected tissue sample while small enough to avoid compromising the structural integrity of the trough portion. When a substantially rectangular opening shape is used, the opening(s) can have a length (relative to the longitudinal axis of the trough portion) ranging from about 0.25 mm to about 0.75 mm. In one embodiment, a rectangular opening has a length of about 0.50 mm. The width (relative to the longitudinal axis of the trough portion) of a rectangular opening can vary as well. The depth (inward direction from a side view relative to the bottom-most portion of the base of the trough) can vary and is typically in the range from about 0.1 mm to about 0.3 mm.

When a plurality of openings are used, the trough portion of the device can comprise combinations of opening shapes having the different sizes, shapes, or both. According to the invention and when a plurality of openings are used, the arrangement of openings 16 in the trough portion 13 can vary as well. Suitable opening arrangements include, but are not limited to, linear alignment along the longitudinal axis of the cannula (as shown in the Figures) and staggered within the trough portion. In a preferred embodiment, a plurality of openings 16 are located within a distance from about 1 cm to about 1.5 cm from the distd end 15 of the trough portion 13.

Referring again to Figure 3, the interior surface 14 of the trough portion 13 further comprises a friction-enhancing surface texture that is adapted to facilitate sample retention. Surface textures which can be used include, but are not limited to, roughened surface textures. When roughened surface texture is used, the surface is roughened to the extent sufficient to increase coefficient of friction of surface to retain sample while at the same time not affording significant resistance to the movement of the tissue into the trough. Various techniques readily available to those skiled in the medical device arts can be used to roughen the interior surface of the trough portion, such as sandblasting and chemical etching. Some or all of the interior surface of the trough portion of the device can be textured.

In a further embodiment, the exterior surface of the proximal portion 11 of the sampling cannula can comprise viewable markings or indicia 17. Markings 17 which can be used include, but are not limited to, orientation indicia, depth markings, numbers, symbols, letters, and the like. Such markings can be printed, etched or embossed. In one particular embodiment and as shown in Figures 1, 2 and 4, the proximal portion 11 of the device comprises external depth markings 17. The markings are viewable upon displacement of the sampling cannula in the proximal direction while the remaining-sampling cannula resides within the outer cannula.

Thus, in use, the distance of displacement of the sampling cannula relative to the outer cannula after the outer cannula has been advanced into the sampling site should substantially correspond to the length of the sample which will be obtained when the sampling cannula is likewise advanced and rotated to sever the sample.

The proximal portion 11 of the sampling cannula of the invention can further comprise a hub 18 coupled thereto in order to facilitate grip and handling by the user during operation of device, e.g., forward pressure for insertion in longitudinal direction and rotational motion for severing sample from site. The hub 18 can be attached to the cannula body 10 using a variety of conventional techniques, such as UV-curable adhesive bonding. The exterior surface of the hub 18 can comprise a surface texture, treatment or geometry to further facilitate grip and handling. The hub 18 can also further comprise markings or indicia, such as an orientation marking 90 (see Figures 1 and 4) indicating the position or alignment of the opening of the trough portion of the sampling cannula.

According to the invention, the sampling cannula can be used as a component of a bone biopsy assembly such as that described by Krueger et al., US. Patent No. 6,443,910. In general, bone biopsy assemblies that can be used which include the sampling cannula of the invention include those such as that shown in Figure 4, which comprises an outer cannula 20 secured or affixed to a handle 22, and a stylet 21 structured to be removably inserted into the outer cannula 20 and which, when inserted into the outer cannula 20 is used to penetrate the cortex of the bone. The outer cannula 20 can be a component of an assembly having a handle 22 secured to the proximal region thereof, and can further comprise a cap 40 which can be positioned over the proximal end of the outer cannula 20 and handle 22 to prevent proximal movement of the stylet 21 during the penetration stage of the procedure.

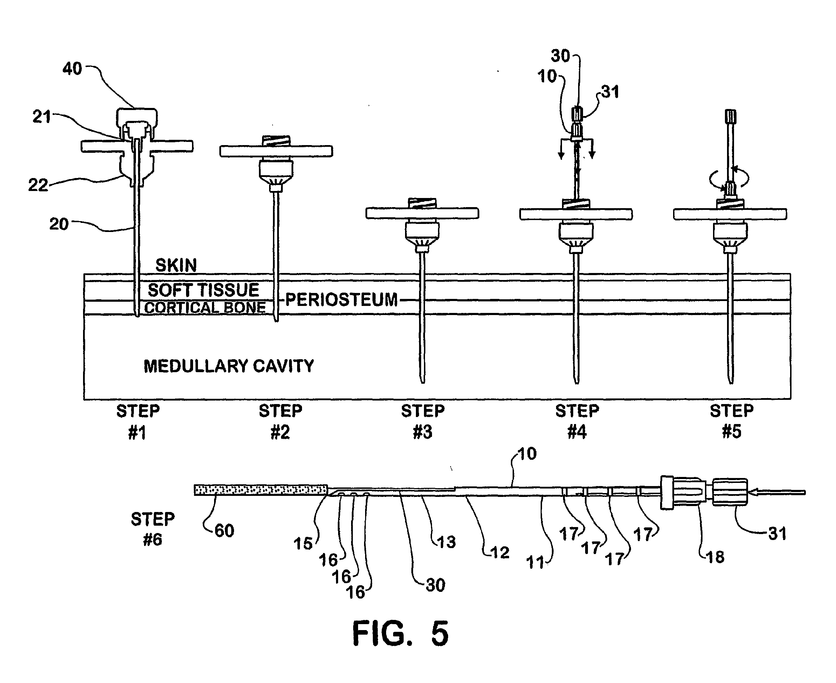

Accordingly, the handle 22 is fixed to the outer cannula 20, and the stylet 21 is positioned within the outer cannula 20 and the cap 40 secured onto the handle 22 and covering the proximal end of the stylet 30. The assembled system is then inserted into the bone as illustrated in Figure 5. When the distal portion of the outer cannula 20 is positioned near the sampling site, the cap 40 is removed and the stylet 21 is withdrawn from the outer cannula 20. The outer cannula 20 is further advanced into the bone to "core" a sample in the longitudinal direction. Subsequently, the sampling cannula of the invention can is inserted into the outer cannula 20 until the distal end of the sampling cannula is positioned just proximal to the distal tip of the outer cannula 20, and then rotated to circumscribe and thus sever the sample from the surrounding tissue. The sampling cannula with the core sample 60 residing within the trough portion 13 is subsequently withdrawn.

The bone biopsy system can further comprise an ejector rod 30 adapted or structured to be inserted within the sampling cannula in order to expel the core sample from the sampling cannula. The ejector rod 30 can comprise an ejector rod hub 31. The ejector rod can be composed of any rigid or semi-rigid material suitable for such use, including polymeric and metallic materials. Preferably, the ejector rod 30 is composed of plastic.

The sampling cannula 10, outer cannula 20 and stylet 21 can be composed of any material which is sterilizable and suitable for use in medical devices and which can withstand the physical forces exerted upon it during bone biopsy techniques. Suitable materials include metals and metallic alloys, such as stainless steel and titanium. The hub, handle and ejector rod components can be composed of polymeric materials or plastic, and made according to conventional molding techniques readily available to those in the medical device field.

### Process of Making the Device:

The following is one example of a manufacturing technique which can be used to make the device in accordance with one embodiment of the invention.

Raw stainless steel tubing is cut to the desired length using a disc cutter to prepare a cannula. A bevel or curved tip is created in one end of the cannula using a grinder at the desired angle relative to the longitudinal axis of the cannula. One or more of the cannulas are fixed onto a plate. A wheel grinder is applied to the uppermost surface of each cannula and applied to the desired depth to remove the uppermost surface of the cannula as well as the desired length of the cannula to be removed. The grinder thus creates a trough structure and exposes the interior of the cannula. Next, a grinding machine is applied to the underside intact portion of the cannula using one or more discs sized and spaced to create the desired dimensions of the openings in the trough portion. More than one opening can be created simultaneously. Electropolishing techniques using electrolytic acid solution can be used to remove burrs and particulate matter from the surface. The exposed interior of the trough portion of the cannula can be sandblasted to create the roughened surface texture. Additionally, the exterior surface of the proximal end of the cannula can be sandblasted as well to facilitate the bonding of a hub thereto. To attach a hub to the proximal end of the cannula, the hub can be positioned on the proximal end and bonding material can be injected into the space between.

Plastic components, for example a sampling cannula hub 18, can be formed in accordance with conventional molding equipment and methods readily available in the art.

### Biopsy Procedure using the Device:

Referring to Figure 4, the following is an example of a bone marrow biopsy procedure using the device according to one embodiment of the invention.

The patient is prepared in accordance with standard surgical preparation techniques for bone biopsy procedures. As seen in Step #1, a bone biopsy assemblyincluding an outer cannula 20 with a removable stylet 21 within and coupled to a handle 22 is inserted into the patient penetrating the skin and cortical layer of the bone to be sampled. Once the cortex of the bone has been penetrated by the distal end of the outer cannula 20, the stylet 21 is then removed as depicted in Step #2. As shown in Step #3, once the stylet has been removed, the outer cannula 20 is further advanced into the sampling site to create a "core" sample within the outer cannula. At this point, the device of the invention is inserted into the interior of the outer cannula to the desired depth as indicated by observing the proximal portion of the device outside the patient's body as depicted in Step #4. Once the device of the invention, specifically the trough portion of the device, has been advanced to the desired extent, the user rotates the hub as shown in Step #5 to rotate the trough portion of the device to sever the sample from the site. The device of the invention is then removed from the bone with the sample contained within the trough. Once outside of the patient's body, the ejector rod 30 can be inserted into the device in a longitudinal direction to expel the sample from the device as shown in Step #6. Alternatively and as shown in Steps # 4 and 5, the ejector rod 30 can accompany the device of the invention throughout the insertion and removal of the device wherein the rod does not penetrate beyond the cortex.

### Industrial Applicability:

The invention is useful in the medical field under circumstances where sampling a patient's bone tissue is needed. The device affords the practitioner or user the advantages of maintaining the architectural.integrity of the sample as well as improved sample retention upon removal.

The invention has been described with reference to various and specific embodiments and techniques. It will be uuderstood, however, that reasonable modifications and variations of such embodiments and techniques can be made.

## Claims

1. A bone marrow biopsy system comprising:
a sampling cannula (10) having a distal trough portion (12, 13) in which the cannula structure is circumferentially incomplete creating an elongated open portion on an opposite side from an intact portion and an exposed interior surface (14) of the sampling cannula viewable through the elongated open portion;
at least one wall opening (16) located within said trough portion located proximal to the distal tip (15) of said cannula;
an outer cannula (20), adapted to core into the bone tissue, **characterized in that**
the sampling cannula is adapted to be inserted into the outer cannula and to sever a sample from a sampling site by rotational motion of the sampling cannula within the outer cannula;
the intact cross-sectional circumference of the trough portion comprises from 65 to 85 % of the complete circumference of the sampling cannula; and **in that** the sampling cannula further comprises a friction-enhancing surface texture on at least a portion of the interior surface of said trough portion.

2. The bone marrow biopsy system according to claim 1, wherein the trough portion (12, 13) comprises a plurality of wall openings (16).

3. The bone marrow biopsy system according to claim 2, wherein the trough portion (12, 13) comprises three wall openings (16).

4. The bone marrow biopsy system according to claim 2, wherein the wall openings (16) have a generally rectangular shape, the longer sides of the rectangle being substantially perpendicular to the longitudinal axis of the sampling cannula (10).

5. The bone marrow biopsy system according to claim 1 further comprising markings (17) located on the exterior surface of the proximal portion (11) of the sampling cannula (10).

6. The bone marrow biopsy system according to claim 5, wherein said markings (17) comprise sampling depth indicia.

7. The bone marrow biopsy system according to claim 1, wherein the proximal portion (11) of the sampling cannula (10) further comprises a hub (18).

8. The bone marrow biopsy system according to claim 7, wherein the sampling cannula hub (18) further comprises orientation indicia (90) relative to the trough portion (12, 13).

9. The bone marrow biopsy system according to claim 1 further comprising a stylet (21) structured to be removably inserted into said outer cannula (20) and wherein the outer cannula has a sharpened distal tip.

10. The bone marrow biopsy system according to claim 9, wherein the distal tip (15) of the trough portion (12, 13) of the sampling cannula (10) resides within the outer cannula (20) such that the trough portion distal tip terminates proximal to the distal tip of the outer cannula.

11. The bone marrow biopsy system according to claim 9 further comprising an ejector rod (30).

12. The bone marrow biopsy system according to claim 11, wherein said ejector rod (30) is composed of plastic.

13. The bone marrow biopsy system according to claim 9, wherein the sampling cannula trough portion (12, 13) comprises a plurality of wall openings (16).

14. The bone marrow biopsy system according to claim 13, wherein the sampling cannula trough portion (12, 13) comprises three wall openings (16).

15. The bone marrow biopsy system according to claim 13, wherein the sampling cannula wall openings (16) have a generally rectangular shape, the longer sides of the rectangle being substantially perpendicular to the longitudinal axis of the sampling cannula (10).

16. The bone marrow biopsy system according to claim 9, wherein the sampling cannula (10) further comprises markings (17) located on the exterior surface of the proximal portion (11) of the sampling cannula.

17. The bone marrow biopsy system according to claim 16, wherein the sampling cannula markings (17) comprise sampling depth indicia that are viewable upon displacement of the sampling cannula (10) in the proximal direction relative to the outer cannula (20).

18. The bone marrow biopsy system according to claim 9, wherein the proximal portion (11) of said sampling cannula (10) further comprises a hub (18).

19. The bone marrow system according to claim 18, wherein the sampling cannula hub (18) further comprises orientation indicia (90) relative to the trough portion.

20. The bone marrow biopsy system according to claim 1 further comprising an ejector rod (30) structured for removable insertion into said sampling cannula (10) to expel a sample residing therein.

21. The bone marrow biopsy system according to claim 20, wherein the proximal portion (11) of said sampling cannula (10) comprises sampling depth indicia (17) that are viewable upon displacement of the sampling cannula in the proximal direction relative to the outer cannula (20).

22. The bone marrow biopsy system according to claim 20, wherein the proximal end of said sampling cannula (10) comprises a hub (18).

23. The bone marrow biopsy system according to claim 22, wherein the hub (18) further comprises orientation indicia (90) relative to the trough portion.

## Patentansprüche

1. Knochenmarkbiopsiesystem, Folgendes umfassend:
eine Probenentnahmekanüle (10) mit einem distalen Rinnenabschnitt (12, 13), in dem die Kanülenanordnung umfänglich unvollständig ist, was einen länglichen offenen Abschnitt auf einer einem intakten Abschnitt gegenüberliegenden Seite und einer freiliegenden Innenoberfläche (14) der Probenentnahmekanüle, die durch den länglichen offenen Abschnitt sichtbar ist, erzeugt;
wenigstens eine Wandöffnung (16), die sich im Rinnenabschnitt befindet, der sich proximal zur distalen Spitze (15) der Kanüle befindet;
eine Außenkanüle (20), angepasst, sich in das Knochengewebe zu bohren,
**dadurch gekennzeichnet, dass** die Probenentnahmekanüle angepasst ist, in die Außenkanüle eingeführt zu werden und eine Probe von einer Probenentnahmestelle durch Drehbewegung der Probenentnahmekanüle in der Außenkanüle abzutrennen;
der intakte Querschnittsumfang des Rinnenabschnitts 65 bis 85 % des vollständigen Umfangs der Probenentnahmekanüle umfasst und dadurch, dass die Probenentnahmekanüle ferner eine reibungserhöhende Oberflächentextur auf wenigstens einem Abschnitt der Innenoberfläche des Rinnenabschnitts umfasst.

2. Knochenmarkbiopsiesystem nach Anspruch 1, wobei der Rinnenabschnitt (12, 13) mehrere Wandöffnungen (16) umfasst.

3. Knochenmarkbiopsiesystem nach Anspruch 2, wobei der Rinnenabschnitt (12, 13) drei Wandöffnungen (16) umfasst.

4. Knochenmarkbiopsiesystem nach Anspruch 2, wobei die Wandöffnungen (16) eine im Allgemeinen rechteckige Form aufweisen, wobei die längeren Seiten des Rechtecks im Wesentlichen senkrecht zur Längsachse der Probenentnahmekanüle (10) sind.

5. Knochenmarkbiopsiesystem nach Anspruch 1, ferner umfassend Markierungen (17), die sich auf der Außenoberfläche des proximalen Abschnitts (11) der Probenentnahmekanüle (10) befinden.

6. Knochenmarkbiopsiesystem nach Anspruch 5, wobei die Markierungen (17) Indikatoren zur Probenentnahmetiefe umfassen.

7. Knochenmarkbiopsiesystem nach Anspruch 1, wobei der proximale Abschnitt (11) der Probenentnahmekanüle (10) ferner eine Nabe (18) umfasst.

8. Knochenmarkbiopsiesystem nach Anspruch 7, wobei die Nabe (18) der Probenentnahmekanüle ferner Ausrichtungsindikatoren (90) bezogen auf den Rinnenabschnitt (12, 13) umfasst.

9. Knochenmarkbiopsiesystem nach Anspruch 1, ferner umfassend ein Mandrin (21), gestaltet, um entfernbar in die Außenkanüle (20) eingeführt zu werden, und wobei die Außenkanüle eine gespitzte distale Spitze aufweist.

10. Knochenmarkbiopsiesystem nach Anspruch 9, wobei sich die distale Spitze (15) des Rinnenabschnitts (12, 13) der Probenentnahmekanüle (10) in der Außenkanüle (20) befindet, sodass die distale Spitze des Rinnenabschnitts proximal zur distalen Spitze der Außenkanüle endet.

11. Knochenmarkbiopsiesystem nach Anspruch 9, ferner umfassend eine Auswerferstange (30).

12. Knochenmarkbiopsiesystem nach Anspruch 11, wobei die Auswerferstange (30) aus Kunststoff besteht.

13. Knochenmarkbiopsiesystem nach Anspruch 9, wobei der Rinnenabschnitt (12, 13) der Probenentnahmekanüle mehrere Wandöffnungen (16) umfasst.

14. Knochenmarkbiopsiesystem nach Anspruch 13, wobei der Rinnenabschnitt (12, 13) der Probenentnahmekanüle drei Wandöffnungen (16) umfasst.

15. Knochenmarkbiopsiesystem nach Anspruch 13, wobei die Wandöffnungen (16) der Probenentnahmekanüle eine im Allgemeinen rechteckige Form aufweisen, wobei die längeren Seiten des Rechtecks im Wesentlichen senkrecht zur Längsachse der Probenentnahmekanüle (10) sind.

16. Knochenmarkbiopsiesystem nach Anspruch 9, wobei die Probenentnahmekanüle (10) ferner Markierungen (17) umfasst, die sich auf der Außenoberfläche des proximalen Abschnitts (11) der Probenentnahmekanüle befinden.

17. Knochenmarkbiopsiesystem nach Anspruch 16, wobei die Markierungen (17) der Probenentnahmekanüle Markierungen zur Probenentnahmetiefe umfassen, die nach der Verschiebung der Probenentnahmekanüle (10) in der proximalen Richtung bezogen auf die Außenkanüle (20) sichtbar sind.

18. Knochenmarkbiopsiesystem nach Anspruch 9, wobei der proximale Abschnitt (11) der Probenentnahmekanüle (10) ferner eine Nabe (18) umfasst.

19. Knochenmarksystem nach Anspruch 18, wobei die Nabe (18) der Probenentnahmekanüle ferner Ausrichtungsindikatoren (90) bezogen auf den Rinnenabschnitt umfasst.

20. Knochenmarkbiopsiesystem nach Anspruch 1, ferner umfassend eine Auswerferstange (30), die zum entfernbaren Einführen in die Probenentnahmekanüle (10) gestaltet ist, um eine darin befindliche Probe auszustoßen.

21. Knochenmarkbiopsiesystem nach Anspruch 20, wobei der proximale Abschnitt (11) der Probenentnahmekanüle (10) Indikatoren (17) zur Probenentnahmetiefe umfasst, die nach der Verschiebung der Probenentnahmekanüle in der proximalen Richtung bezogen auf die Außenkanüle (20) sichtbar sind.

22. Knochenmarkbiopsiesystem nach Anspruch 20, wobei das proximale Ende der Probenentnahmekanüle (10) eine Nabe (18) umfasst.

23. Knochenmarkbiopsiesystem nach Anspruch 22, wobei die Nabe (18) ferner Ausrichtungsindikatoren (90) bezogen auf den Rinnenabschnitt umfasst.

## Revendications

1. Système de biopsie de moelle osseuse comprenant :
une canule de prélèvement (10) ayant une partie de gouttière distale (12, 13) dans laquelle la structure de canule est circonférentiellement incomplète, créant une partie ouverte allongée sur un côté opposé par rapport à une partie intacte et une surface intérieure exposée (14) de la canule de prélèvement pouvant être vue à travers la partie ouverte allongée ;
au moins une ouverture de paroi (16) située à l'intérieur de ladite partie de gouttière proximale à la pointe distale (15) de ladite canule ;
une canule externe (20), adaptée pour s'introduire dans le tissu osseux, **caractérisé en ce que**
la canule de prélèvement est adaptée pour être insérée dans la canule externe et pour séparer un échantillon d'un site de prélèvement par un mouvement rotatif de la canule de prélèvement à l'intérieur de la canule externe ;
la circonférence en coupe intacte de la partie de gouttière comprend de 65 à 85 % de la circonférence entière de la canule de prélèvement ; et **en ce que** la canule de prélèvement comprend en outre
une texture de surface améliorant le frottement sur au moins une partie de la surface intérieure de ladite partie de gouttière.

2. Système de biopsie de moelle osseuse selon la revendication 1, dans lequel la partie de gouttière (12, 13) comprend une pluralité d'ouvertures de paroi (16).

3. Système de biopsie de moelle osseuse selon la revendication 2, dans lequel la partie de gouttière (12, 13) comprend trois ouvertures de paroi (16).

4. Système de biopsie de moelle osseuse selon la revendication 2, dans lequel les ouvertures de paroi (16) ont une forme généralement rectangulaire, les côtés plus longs du rectangle étant sensiblement perpendiculaires à l'axe longitudinal de la canule de prélèvement (10).

5. Système de biopsie de moelle osseuse selon la revendication 1, comprenant en outre des repères (17) situés sur la surface extérieure de la partie proximale (11) de la canule de prélèvement (10).

6. Système de biopsie de moelle osseuse selon la revendication 5, dans lequel lesdits repères (17) comprennent des indications de profondeur de prélèvement.

7. Système de biopsie de moelle osseuse selon la revendication 1, dans lequel la partie proximale (11) de la canule de prélèvement (10) comprend en outre un raccord (18).

8. Système de biopsie de moelle osseuse selon la revendication 7, dans lequel le raccord de canule de prélèvement (18) comprend en outre une indication d'orientation (90) par rapport à la partie de gouttière (12, 13).

9. Système de biopsie de moelle osseuse selon la revendication 1, comprenant en outre un stylet (21) structuré pour être inséré de façon amovible dans ladite canule externe (20) et dans lequel la canule externe a une pointe distale aiguisée.

10. Système de biopsie de moelle osseuse selon la revendication 9, dans lequel la pointe distale (15) de la partie de gouttière (12, 13) de la canule de prélèvement (10) est située à l'intérieur de la canule externe (20) de sorte que la pointe distale de la partie de gouttière se termine proximale à la pointe distale de la canule externe.

11. Système de biopsie de moelle osseuse selon la revendication 9, comprenant en outre une tige d'éjection (30).

12. Système de biopsie de moelle osseuse selon la revendication 11, dans lequel ladite tige d'éjection (30) est composée de plastique.

13. Système de biopsie de moelle osseuse selon la revendication 9, dans lequel la partie de gouttière de canule de prélèvement (12, 13) comprend une pluralité d'ouvertures de paroi (16).

14. Système de biopsie de moelle osseuse selon la revendication 13, dans lequel la partie de gouttière de canule de prélèvement (12, 13) comprend trois ouvertures de paroi (16).

15. Système de biopsie de moelle osseuse selon la revendication 13, dans lequel les ouvertures de paroi de canule de prélèvement (16) ont une forme généralement rectangulaire, les côtés plus longs du rectangle étant sensiblement perpendiculaires à l'axe longitudinal de la canule de prélèvement (10).

16. Système de biopsie de moelle osseuse selon la revendication 9, dans lequel la canule de prélèvement (10) comprend en outre des repères (17) situés sur la surface extérieure de la partie proximale (11) de la canule de prélèvement.

17. Système de biopsie de moelle osseuse selon la revendication 16, dans lequel les repères de canule de prélèvement (17) comprennent des indications de profondeur de prélèvement qui peuvent être vues lors du déplacement de la canule de prélèvement (10) dans la direction proximale par rapport à la canule externe (20).

18. Système de biopsie de moelle osseuse selon la revendication 9, dans lequel la partie proximale (11) de ladite canule de prélèvement (10) comprend en outre un raccord (18).

19. Système de moelle osseuse selon la revendication 18, dans lequel le raccord de canule de prélèvement (18) comprend en outre des indications d'orientation (90) par rapport à la partie de gouttière.

20. Système de biopsie de moelle osseuse selon la revendication 1, comprenant en outre une tige d'éjection (30) structurée pour une insertion amovible dans ladite canule de prélèvement (10) afin d'expulser un échantillon y étant situé.

21. Système de biopsie de moelle osseuse selon la revendication 20, dans lequel la partie proximale (11) de ladite canule de prélèvement (10) comprend des indications de profondeur de prélèvement (17) qui peuvent être vues lors du déplacement de la canule de prélèvement dans la direction proximale par rapport à la canule externe (20).

22. Système de biopsie de moelle osseuse selon la revendication 20, dans lequel l'extrémité proximale de ladite canule de prélèvement (10) comprend un raccord (18).

23. Système de biopsie de moelle osseuse selon la revendication 22, dans lequel le raccord (18) comprend en outre des indications d'orientation (90) par rapport à la partie de gouttière.
